# EUROPÄISCHE PATENTSCHRIFT

(11) **EP 3 594 966 B1**
(45) Veröffentlichungstag und Bekanntmachung des Hinweises auf die Patenterteilung: **16.03.2022**
(21) Anmeldenummer: 19181982.0
(22) Anmeldetag: 24.06.2019
(51) Int. Cl.: G21F 9/00, A61L 2/22, B08B 3/02, B60S 3/00, B08B 17/02, B60S 3/04

(54) **MOBILES DEKONTAMINATIONSSYSTEM ZUM ENTSTRAHLEN, ENTSEUCHEN ODER ENTGIFTEN VON FAHRZEUGEN**
MOBILE DECONTAMINATION SYSTEM FOR DECONTAMINATING VEHICLES
SYSTÈME MOBILE DE DÉCONTAMINATION PERMETTANT DE STÉRILISER, DE DÉSINFECTER ET DE DÉSINTOXIQUER DES VÉHICULES

(30) Priorität: 09.07.2018 DE 102018116546
(43) Veröffentlichungstag der Anmeldung: 15.01.2020
(73) Patentinhaber: Kärcher Futuretech GmbH, 71409 Schwaikheim (DE)
(72) Erfinder: Schweizer, Stefan, 71522 Backnang (DE); Spaan, Michael, 71106 Magstadt (DE)
(74) Vertreter: Witte, Weller & Partner Patentanwälte mbB

(56) Entgegenhaltungen:
- AU-A1- 2006 202 061
- DE-U1- 9 302 653
- US-A1- 2005 066 997
- US-A1- 2005 214 159
- US-B2- 9 770 523

## Beschreibung

Die vorliegende Erfindung betrifft ein mobiles Dekontaminationssystem zum Entstrahlen, Entseuchen oder Entgiften von Fahrzeugen, mit zumindest einer Sprühvorrichtung zum gezielten Austragen eines zumindest teilweise flüssigen Dekontaminationsmittels auf das Fahrzeug, und mit einer Steuereinrichtung, die dazu eingerichtet ist, die zumindest eine Sprühvorrichtung in Abhängigkeit von dem zu dekontaminierenden Fahrzeug automatisiert zu betreiben.

Ein solches Dekontaminationssystem ist aus US 9,770,523 B2 bekannt.

CN 2885261 Y offenbart ein Dekontaminationssystem mit einer zweiteiligen Wanne mit einem ersten Wannenteil und einem zweiten Wannenteil, die in Längsrichtung parallel nebeneinander und mit einem individuell einstellbaren Abstand voneinander angeordnet werden können. Ein zu dekontaminierendes Fahrzeug steht mit den linken Rädern in dem ersten Wannenteil und mit den rechten Rädern in dem zweiten Wannenteil. Ein Gestell mit einer Vielzahl von Sprühdüsen bildet ein Portal, durch das ein zu dekontaminierendes Fahrzeug in der Wanne hindurch fährt. Die Portalhöhe kann an unterschiedliche Fahrzeughöhen und die Portalbreite an unterschiedlichen Fahrzeugbreiten angepasst werden. Das von dem Fahrzeug ablaufende Dekontaminationsmittel sammelt sich in den beiden Wannenteilen und kann von dort in Entsorgungstanks abgesaugt werden. Alle Komponenten des bekannten Dekontaminationssystems können mit einem Lastwagen zu einem Einsatzort transportiert werden. Das bekannte Dekontaminationssystem bietet damit die Möglichkeit, Fahrzeuge beim Verlassen eines mit Krankheitserregern verseuchten Gebiets zu entseuchen.

Ein weiteres mobiles Dekontaminationssystem ist in DE 44 01 695 C1 offenbart. Dieses bekannte Dekontaminationssystem besitzt eine bogenförmige Rohrdüse, die an einem Tragarm angeordnet ist. Zur Dekontamination eines Fahrzeugs wird die Rohrdüse mithilfe eines Schlittens portalartig über das zu dekontaminierende Fahrzeug bewegt. Eine Wanne zum Aufsammeln des von dem Fahrzeug ablaufenden Dekontaminationsmittels ist ebenso wenig vorgesehen wie eine Unterbodendekontamination.

DE 102 08 927 A1 offenbart ein weiteres mobiles Dekontaminationssystem für Fahrzeuge. Dieses bekannte System besitzt eine Wanne, einen aus mehreren Segmenten zusammengesetzten portalartigen Rahmen, an dem eine Vielzahl von Sprühdüsen angeordnet sind, und ein in der Wanne angeordnetes Bodensprührohr zum Dekontaminieren eines Fahrzeugunterbodens. Eine Entsorgung des von einem Fahrzeug ablaufenden Dekontaminationsmittels ist hier nicht thematisiert.

Die eingangs genannte US 9,770,523 B2 offenbart ein weiteres Dekontaminationssystem zum Dekontaminieren von Fahrzeugen. Dieses bekannte System besitzt ein Portal, das auf zwei Schienen in einer Längsrichtung bewegbar ist. An dem Portal sind bewegliche Sprühlanzen angeordnet, die in Abhängigkeit von der Kontur des zu dekontaminierenden Fahrzeugs eingestellt werden können. In einem Ausführungsbeispiel kann die Dekontamination in einer Halle oder einem Zelt stattfinden, in denen das Portal und die Führungsschienen aufgebaut sind.

AU 2006202061 A1 und EP 0 363 751 A2 offenbaren jeweils mobile Wannen, um das bei einer normalen Autowäsche anfallende Schmutzwasser aufzusammeln und gegebenenfalls einer Entsorgung oder Wiederaufbereitung zuzuführen. Die Fahrzeugwäsche erfolgt hier regelmäßig von Hand und berücksichtigt nicht die Gefahren und Herausforderungen beim Entstrahlen, Entseuchen oder Entgiften von Fahrzeugen.

US 2005/0214159 A1 offenbart ein System zum Desinfizieren von Einkaufswagen. Das System beinhaltet eine Umhausung mit einer Einfahrrampe und einer Ausfahrrampe, über die zu desinfizierende Einkaufswagen durch die Umhausung hindurch geschoben werden können. In der Umhausung sind an einem portalartigen Träger und am Boden Sprühdüsen angeordnet, über die ein Desinfektionsmittel versprüht werden kann. Ein Ablauf für das Desinfektionsmittel befindet sich am Boden im Bereich der Ausfahrrampe.

US 2005/0066997 A1 offenbart eine mobile Waschanlage für Fahrzeuge. Die Waschanlage beinhaltet einen Container, der auf einem Lastwagen transportiert werden kann. Das Fahrzeug steht während des Waschvorgangs auf einem Boden mit Ablauföffnungen, durch die das vom Fahrzeug ablaufende Wasser in eine Sammelwanne unterhalb des Waschbereichs tropft. Alternativ hierzu könnte der Boden des Containers leicht geneigt sein, um das ablaufende Wasser der Sammelwanne zuzuführen.

DE 93 02 653 U1 offenbart eine Waschanlage zum Reinigen von Fahrzeugreifen, insbesondere von Lkw-Reifen von Baustellenfahrzeugen. Die Waschanlage beinhaltet ein als Wanne ausgebildetes Auffangbecken. In dem Auffangbecken sind Rollen angeordnet, auf denen die Fahrzeugreifen abrollen können, während sie von Sprühdüsen mit Wasser besprüht werden. Am tiefsten Punkt des Auffangbeckens ist ein Schneckenförderer angeordnet, der feste Partikel aus dem Auffangbecken herausbefördert. Über einen höher gelegenen Ablauf kann das Wasser aus dem Auffangbecken in ein Sammelbecken überlaufen. In dem Sammelbecken ist ein Rührwerk vorhanden, das im Sammelbecken eine permanente Strömung aufrechterhält.

Generell besitzen die bekannten Systeme den Nachteil, dass ein mehr oder minder großer manueller Aufwand für die Dekontamination erforderlich ist. Dies gilt insbesondere dann, wenn die zu dekontaminierenden Fahrzeuge zusätzlich zu der gefährlichen Kontamination sehr stark verschmutzt sind, was beispielsweise nach einem militärischen Einsatz in einem verstrahlten, verseuchten oder vergifteten Gebiet nach starken Regenfällen der Fall sein kann. Große Schlammmengen, Gesteinsbrocken und andere Verunreinigungen erschweren die Entsorgung des kontaminierten Abwassers und erhöhen die Gefahr von Kontaminationsverschleppungen. Beispielsweise kann eine Kontamination leicht verschleppt werden, wenn bereits dekontaminierte Fahrzeugbereiche wieder mit dem kontaminierten Abwasser in der Wanne in Berührung kommen. Eine schnelle Absaugung ist bei großen Schlammmengen, Gesteinsbrocken, Pflanzenteilen und anderen Verunreinigungen mit den bekannten Systemen nicht ohne weiteres möglich.

Ebenso ist eine Kontaminationsverschleppung von einem kontaminierten Fahrzeug zu einem nachfolgenden Fahrzeug nur ausgeschlossen, wenn die Wanne nach der Dekontamination des ersten Fahrzeugs vollständig entleert wird, bevor das nachfolgende Fahrzeug in die Wanne einfährt. Eine vollständige Entleerung vor dem Einfahren des nächsten Fahrzeugs kostet allerdings viel Zeit und erfordert häufig körperliche Arbeit der beteiligten Personen. Dies birgt unter Einsatzbedingungen das Risiko menschlichen Versagens.

Häufig werden Fahrzeuge, die aus einem Einsatz in einem kontaminierten Gebiet kommen, auch heute noch manuell dekontaminiert, was eine sehr schwere körperliche Arbeit darstellt, da die Personen, die die Dekontamination durchführen, unter Vollschutz arbeiten. Eine manuelle Dekontamination birgt generell das Risiko, dass kontaminierte Fahrzeugbereiche aufgrund von Konzentrationsmängeln, Erschöpfung oder dergleichen übersehen werden.

Wünschenswert ist daher ein mobiles Dekontaminationssystem, das eine weitgehend automatisierte Dekontamination von Fahrzeugen ermöglicht und Kontaminationsverschleppungen systematisch entgegenwirkt. Dementsprechend ist es eine Aufgabe der vorliegenden Erfindung, ein solches Dekontaminationssystem anzugeben.

Gemäß einem Aspekt der vorliegenden Erfindung wird diese Aufgabe durch ein mobiles Dekontaminationssystem mit den Merkmalen von Anspruch 1 gelöst.

Die Wanne des neuen Dekontaminationssystems besitzt demnach über den Behandlungsbereich für das Fahrzeug hinaus einen definierten Entwässerungsbereich, dem das in der Wanne anfallende Abwasser einschließlich des von dem zu dekontaminierenden Fahrzeug ablaufenden Dekontaminationsmittels zielgerichtet zugeführt wird. Das Dekontaminationsmittel kann Wasser mit oder ohne zusätzlichen Reinigungsmitteln und/oder chemischen Mitteln zum Entgiften, Entseuchen oder Entstrahlen sein.

Der definierte Entwässerungsbereich ist einerseits ein Teil der Wanne. Andererseits ist er von dem Behandlungsbereich, in dem sich der Gegenstand während der Dekontamination befindet, zu unterscheiden. Der definierte Entwässerungsbereich ist somit ein zusätzlicher Wannenbereich, der speziell dafür vorgesehen ist, das Abwasser schon während der Dekontamination aus dem Behandlungsbereich abzuführen. Vorzugsweise ist der definierte Entwässerungsbereich exklusiv in der Nähe des Anfangsbereichs angeordnet, was den Vorteil besitzt, dass das kontaminierte Abwasser allein auf der schmutzigen bzw. schwarzen Seite des Dekontaminationssystems gesammelt wird. Dies trägt besonders vorteilhaft dazu bei, Kontaminationsverschleppungen wirksam zu vermeiden.

Bevorzugt erfolgt die Entleerung des definierten Entwässerungsbereichs schon vor dem Einfahren mit Hilfe der Steuereinrichtung und einer geeigneten Pumpe vollautomatisch. Des Weiteren ist es bevorzugt, wenn die Entleerung des definierten Entwässerungsbereichs während der Dekontamination durchgehend erfolgt.

In allen Ausführungsbeispielen besitzt die Wanne ein in Längsrichtung gesehen erstes Ende, an dem ein zu dekontaminierendes Fahrzeug in die Wanne hineinfahren kann, sowie ein in Längsrichtung gesehen zweites Ende, an dem es die Wanne wieder verlässt. Der Behandlungsbereich, in dem sich der Gegenstand während der Dekontamination befindet, liegt zwischen dem ersten und dem zweiten Ende und der Entwässerungsbereich liegt - vorzugsweise exklusiv - an dem ersten Ende und ist somit von dem Behandlungsbereich separiert.

Darüber hinaus besitzt das neue Dekontaminationssystem eine Anordnung, die es möglich macht, kontaminiertes Abwasser kontrolliert aus dem Behandlungsbereich in den definierten Entwässerungsbereich zu befördern. Vorteilhaft besitzt das neue Dekontaminationssystem eine oder mehrere Pumpen, mit denen kontaminiertes Abwasser aus dem definierten Entwässerungsbereich kontrolliert abgesaugt werden kann. In einigen Ausführungsbeispielen können ein oder mehrere Rotoren, ein oder mehrere Sprühdüsen und/oder andere Rührwerkzeuge in dem definierten Entwässerungsbereich angeordnet sein, um das dort gesammelte Abwasser aufzurühren und insbesondere große Schlamm- oder Matschklumpen zu zerteilen, um eine effiziente Absaugung zu erleichtern. Die Sprühdüsen können als Wassersprühdüsen und/oder Pressluftdüsen ausgebildet sein. Vorzugsweise ist die Anordnung in der Lage, Feststoffe in dem kontaminierten Abwasser, wie Matsch, Schlamm, Steine, Geröll, Pflanzenreste u.a. von wässerigen/flüssigen Anteilen zu trennen, um eine effiziente Absaugung zu ermöglichen.

In den bevorzugten Ausführungsbeispielen ist die genannte Anordnung dazu eingerichtet, das Abwasser mit Hilfe der Steuereinrichtung selektiv, d.h. aktiv gesteuert aus dem Behandlungsbereich in den definierten Entwässerungsbereich zu befördern, so dass das Abwasser zu einem definierten Zeitpunkt während eines Dekontaminationsprozesses aus dem Behandlungsbereich entfernt wird. Alternativ oder ergänzend kann die Anordnung in weiteren Ausführungsbeispielen passiv wirken, beispielsweise unter Ausnutzung der Schwerkraft.

In jedem Fall wird die Entleerung des Behandlungsbereichs mit dem neuen Dekontaminationssystem erleichtert und beschleunigt. Daher trägt das neue Dekontaminationssystem vorteilhaft dazu bei, Kontaminationsverschleppungen wirkungsvoll zu reduzieren, wenn nicht gar gänzlich zu vermeiden.

Vorteilhaft ist der definierte Entwässerungsbereich seitlich von dem Anfangsbereich angeordnet.

Diese Ausgestaltung ermöglicht eine einfache und kostengünstige Integration eines großen definierten Entwässerungsbereichs, in dem sich große Schlammklumpen sammeln können, in die Wanne. Zugleich besitzt diese Ausgestaltung den Vorteil, dass ein zu dekontaminierendes Fahrzeug an dem Entwässerungsbereich vorbei in die Wanne einfahren kann, was vorteilhaft dazu beiträgt, den Durchsatz zu erhöhen und Kontaminationsverschleppungen zu vermeiden.

In einer weiteren Ausgestaltung weist die Anordnung einen Wellenerzeuger auf, der dazu eingerichtet ist, das in der Wanne gesammelte Dekontaminationsmittel in eine Wellenbewegung mit einer definierten Wellenlaufrichtung zu versetzen, wobei die definierte Wellenlaufrichtung zu dem Entwässerungsbereich gerichtet ist.

In dieser Ausgestaltung wird das Abwasser mithilfe einer künstlich erzeugten dynamischen Wellenbewegung zu dem definierten Entwässerungsbereich befördert. Die Ausgestaltung besitzt den Vorteil, dass Schlammklumpen aufgrund der dynamischen Wellenbewegung zerteilt oder abgetragen werden und so wirksam zu dem Entwässerungsbereich befördert werden. Außerdem trägt eine Wellenbewegung vorteilhaft dazu bei, das in dem Behandlungsbereich gesammelte Abwasser großflächig zu dem Entwässerungsbereich zu befördern.

In einer weiteren Ausgestaltung weist der Wellenerzeuger zumindest ein Fluidkissen auf, das in oder unter der Wanne angeordnet ist, und eine Fluidpumpe, die dazu eingerichtet ist, das zumindest eine Fluidkissen wahlweise aufzupumpen und/oder zu entleeren. Vorzugsweise ist die Steuereinrichtung dazu eingerichtet, die Fluidpumpe intervallweise anzusteuern, um das Fluidkissen stoßweise aufzupumpen und/oder zu entleeren.

Diese Ausgestaltung ermöglicht eine einfache und robuste Realisierung des Wellenerzeugers. Fluidkissen können nach ihrer Entleerung zudem klein verpackt werden. In einigen Ausführungsbeispielen ist der Wannenboden eine - vorzugsweise einteilige - Plane mit angeschweißten und/oder integrierten aufblasbaren Seitenwülsten, die einen Wannenrand bilden. In diesen Ausführungsbeispielen ist die Realisierung des Wellenerzeugers sehr kostengünstig möglich, da das Kissen auf dieselbe Weise aufgeblasen werden kann wie der Wannenrand.. Das zumindest eine Fluidkissen kann in den Wannenboden integriert sein. Alternativ oder ergänzend kann das zumindest Fluidkissen als separates Fluidkissen in die Wanne eingelegt werden, was einen einfacheren Austausch im Fall einer Beschädigung des Fluidkissens ermöglicht.

In einer weiteren Ausgestaltung weist der Wellenerzeuger eine Vielzahl an Fluidkissen auf, die in einer Reihe zueinander angeordnet sind. Vorzugsweise erstreckt sich die Reihe in der Längsrichtung der Wanne.

In dieser Ausgestaltung erzeugt der Wellenerzeuger eine Transversalwelle, deren Wellenlaufrichtung und Wellenperiode durch sequentielle Befüllung und Entlüftung der Fluidkissen definiert werden kann. Außerdem kann der Ort der Wellenentstehung variiert werden, was vorteilhaft dazu genutzt werden kann, um Schlammklumpen, Steine, Geröll u.a. gezielt zu dem Entwässerungsbereich zu befördern.

In einer weiteren Ausgestaltung weist der Wellenerzeuger einen Motor auf, der ein Oszillationselement antreibt. Das Oszillationselement kann ein Balken, eine Platte, ein Gitter, eine Anzahl Finger oder ein anderes in die Wanne und insbesondere in das Abwasser hineinragendes Element sein, das mithilfe des Motors zyklisch bewegt wird. In einigen Ausführungsbeispielen führt das Oszillationselement eine translatorische Hin- und Herbewegung aus, um die Wellenbewegung zu erzeugen. In anderen Ausführungsbeispielen kann das Oszillationselement einen Exzenter beinhalten, der aus einer Rotationsbewegung die oszillierende Bewegung erzeugt.

In dieser Ausgestaltung erzeugt der Wellenerzeuger im Wesentlichen eine Longitudinalwelle, mit der das Abwasser sehr effizient aus dem Behandlungsbereich in den Entwässerungsbereich befördert werden kann. Vorzugsweise ist das Oszillationselement in dieser Ausgestaltung an dem zweiten Ende der Wanne angeordnet, was eine sehr platzsparende Realisierung ermöglicht.

In einer weiteren Ausgestaltung weist die Anordnung Strahldüsen auf, die dazu eingerichtet sind, das gesammelte Dekontaminationsmittel mithilfe von Fluidstrahlen, wie etwa Wasserstrahlen und/oder Pressluftstrahlen, dem definierten Entwässerungsbereich zuzuführen.

In einigen Varianten dieser Ausgestaltung tragen die Strahldüsen dazu bei, die definierte Wellenbewegung des Abwassers zu erzeugen, indem die Steuereinrichtung beispielsweise dazu eingerichtet ist, die die Strahldüsen impulsartig und zyklisch anzusteuern. Vorteilhaft kann gefiltertes oder ungefiltertes Brauchwasser zum Erzeugen der Wasserstrahlen verwendet werden. In weiteren Varianten unterstützen die Strahldüsen eine mithilfe eines Wellenerzeugers erzeugte Wellenbewegung, um das Abwasser noch effizienter und schneller aus dem Behandlungsbereich heraus zu befördern.

In einer weiteren Ausgestaltung weist die Wanne einen Wannenboden auf und die Anordnung weist eine Keilstruktur auf, die dazu eingerichtet ist, den Wannenboden mit einer definierten Ablaufrichtung zu dem definierten Entwässerungsbereich schräg zu stellen.

Dieser Ausgestaltung ist besonders vorteilhaft in Kombination mit einem aktiven Wellenerzeuger und/oder einer aktiv gesteuerten Anordnung mit Strahldüsen, um das Abwasser einschließlich großer Schlammklumpen schnell und effizient in den Entwässerungsbereich zu befördern.

In einer weiteren Ausgestaltung ist die Steuereinrichtung ferner dazu eingerichtet, das Dekontaminationsmittel in der Wanne dem definierten Entwässerungsbereich automatisiert zuzuführen. In einigen Ausführungsbeispielen erzeugt die Steuereinrichtung mithilfe des Wellenerzeugers und/oder mithilfe der Strahldüsen eine gerichtete Transportströmung, während das zu dekontaminierende Fahrzeug in der Wanne steht und/oder nachdem ein dekontaminiertes Fahrzeug die Wanne verlassen hat, nicht jedoch während ein Fahrzeug in die Wanne ein- oder ausfährt.

Die Ausgestaltung trägt vorteilhaft dazu bei, einen kontrollierten und wiederholbaren Dekontaminationsprozess zu implementieren, bei dem die Einhaltung vorgegebener Prozessparameter gewährleistet ist. Die Ausgestaltung trägt daher vorteilhaft dazu bei, einen Dekontaminationserfolg zu gewährleisten.

In einer weiteren Ausgestaltung weist die zumindest eine Sprühvorrichtung ein fahrbares Portal mit zwei Säulen auf, die jeweils auf einem Laufwagen abgestützt sind. In bevorzugten Ausführungsbeispielen ist das Portal in der Höhe und in der Breite verstellbar und die Steuereinrichtung ist dazu eingerichtet, das Portal an die Fahrzeugkontur des zu dekontaminierenden Fahrzeugs anzupassen. Des Weiteren besitzt das Portal in bevorzugten Ausführungsbeispielen schwenkbare Sprühdüsen zum Austragen des Dekontaminationsmittels, wie etwa Wasser oder wässrige Lösungen mit chemischen Zusätzen zum Reinigen und/oder Entgiften, Entseuchen oder Entstrahlen, wobei die Steuereinrichtung die schwenkbaren Düsen in einem automatisierten Dekontaminationsprozess ansteuert.

Ein fahrbares Portal mit zwei auf einem Laufwagen abgestützten Säulen ermöglicht einen vollautomatischen Dekontaminationsprozess mit einer definierten Relativgeschwindigkeit zwischen den Sprühdüsen und dem Fahrzeug. Die Ausgestaltung trägt vorteilhaft dazu bei, individuelle menschliche Fehler zu vermeiden und so einen Dekontaminationserfolg auf kostengünstige Weise zu gewährleisten.

In einer weiteren Ausgestaltung weist die Unterbodensprühvorrichtung zumindest eine Unterbodensprühdüse auf, die in vertikaler Richtung verstellbar ist, um einen definierten Sprühabstand zwischen der zumindest einen Sprühdüse und einem Fahrzeugunterboden einzustellen. In bevorzugten Ausführungsbeispielen besitzt die Unterbodensprühvorrichtung ein Unterbodenabtastelement, um einen Abstand zwischen dem Unterboden des zu dekontaminierenden Fahrzeugs und der Unterbodensprühdüse zu bestimmen. Das Unterbodenabtastelement kann in einigen Ausführungsbeispielen ein taktiles Abtastelement sein, wie etwa ein Rollrad, das mit einem Federmechanismus gekoppelt ist. Der Federmechanismus spannt das Rollrad in vertikaler Richtung nach oben gegen den Unterboden des Fahrzeugs vor. In anderen Ausführungsbeispielen kann das Unterbodenabtastelement ein berührungsloser Sensor sein, wie etwa ein Laserscanner, eine Lichtschranke, ein Lichtgitter oder ein induktiver, kapazitiver oder optischer Distanzsensor. In diesen Ausführungsbeispielen kann die Unterbodensprühvorrichtung vorteilhaft mithilfe eines Elektromotors in vertikaler Richtung bewegt werden, wobei die Steuereinrichtung dazu eingerichtet ist, die zumindest eine Sprühdüse in einem definierten Sprühabstand zu dem Unterboden zu halten. Des Weiteren kann die Unterbodensprühvorrichtung ein Federelement beinhalten, das so dimensioniert ist, dass die zumindest eine Unterbodensprühdüse aufgrund des Rückstoßprinzips actio=reactio, d.h. also aufgrund der Rückstoßkraft des austretenden Fluids, in einem definierten Abstand zu dem Unterboden gehalten wird.

In den bevorzugten Ausführungsbeispielen besitzt die Unterbodensprühvorrichtung eine Vielzahl von Unterbodensprühdüsen, die individuell oder gruppenweise verstellbar sind, um den Fahrzeugunterboden abschnittsweise mit jeweils einem definierten und vorzugsweise weitgehend konstanten Sprühabstand zu dekontaminieren.

Diese Ausgestaltung, die auch für sich genommen eine vorteilhafte Weiterentwicklung gegenüber bekannten Dekontaminationssystemen darstellt, trägt zu einer effizienten und zuverlässigen Dekontamination unterschiedlicher Fahrzeuge und Fahrzeugtypen bei. Dies ist besonders vorteilhaft in Kombination mit dem oben beschriebenen Entwässerungsbereich, da im Behandlungsbereich gesammeltes Abwasser durch die Unterbodensprühdüsen und/oder vom Unterboden herabfallende Schlammklumpen hochgeschleudert werden kann und so zu einer Rekontamination des Fahrzeugunterbodens führen kann. Eine gezielte Ableitung des kontaminierten Abwassers aus dem Behandlungsbereich in Kombination mit der hier beschriebenen Unterbodensprühvorrichtung trägt daher wirkungsvoll dazu bei, einen Erfolg des Dekontaminationsprozesses zu gewährleisten und Kontaminationsverschleppungen zu vermeiden.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein Ausführungsbeispiel des neuen Dekontaminationssystems mit einem fahrbaren Portal, wobei hier aus Gründen der Übersichtlichkeit die Wanne nicht dargestellt ist,
- Fig. 2: das Portal des Dekontaminationssystems aus Fig. 1 nach Verladung in einem Transportcontainer,
- Fig. 3: ein zu dekontaminierendes Fahrzeug beim Einfahren in die Wanne des neuen Dekontaminationssystems gemäß Fig. 1, wobei hier das Portal aus Gründen der Übersichtlichkeit nicht dargestellt ist,
- Fig. 4: das Fahrzeug aus Fig. 3, nachdem es weiter in die Wanne hineingefahren ist,
- Fig. 5: eine Wanne gemäß einem weiteren Ausführungsbeispiel des neuen Dekontaminationssystems,
- Fig. 6: eine Wanne gemäß einem weiteren Ausführungsbeispiel des neuen Dekontaminationssystems,
- Fig. 7: ein Ausführungsbeispiel der Unterbodensprühvorrichtung in einer ersten Betriebsstellung, und
- Fig. 8: das Ausführungsbeispiel aus Fig. 7 in einer zweiten Betriebsstellung.

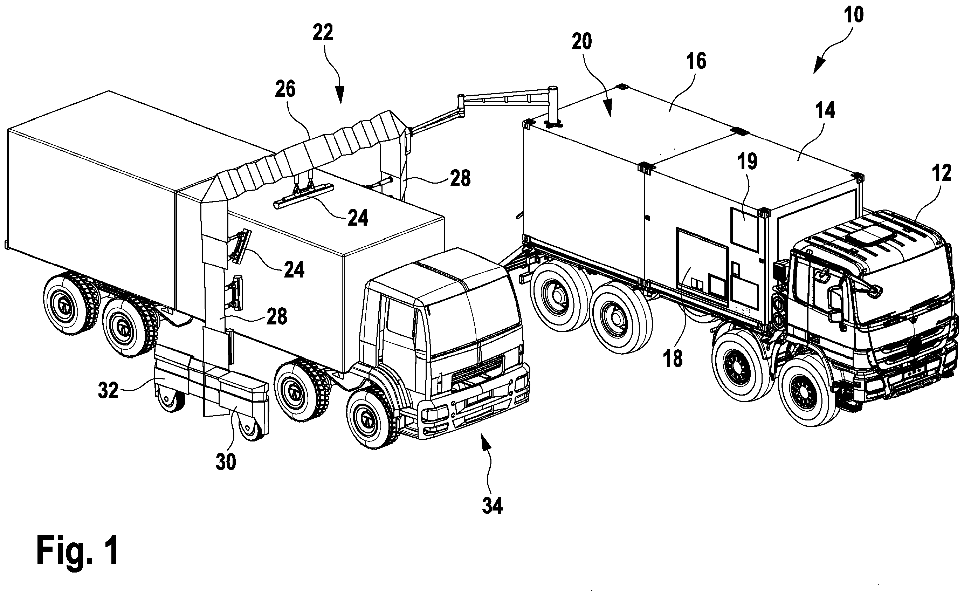

In den Fig. 1 bis 4 ist ein Ausführungsbeispiel des neuen Dekontaminationssystems in seiner Gesamtheit mit der Bezugsziffer 10 bezeichnet, wobei die zu dem Dekontaminationssystem 10 gehörende Wanne in Fig. 1 aus Gründen der Übersichtlichkeit nicht dargestellt ist.

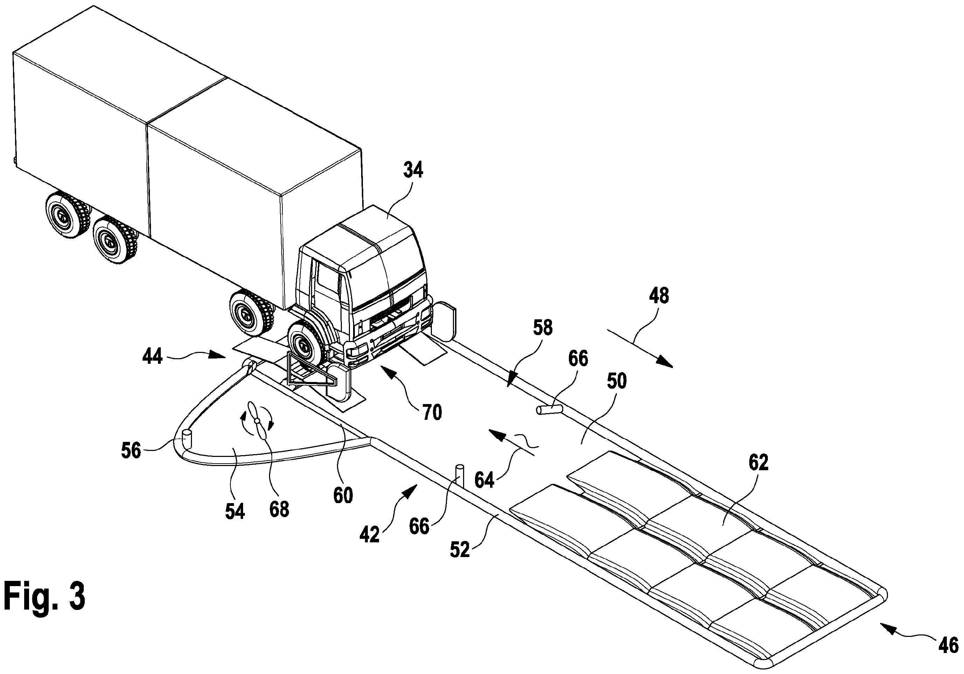

Das Dekontaminationssystem 10 beinhaltet in diesem Ausführungsbeispiel ein Transportfahrzeug 12, auf dem hier zwei Transportcontainer 14, 16 befestigt sind. In den bevorzugten Ausführungsbeispielen sind die Transportcontainer 14, 16 beispielsweise Seefrachtcontainer gemäß ISO 668. In dem dargestellten Ausführungsbeispiel beherbergt der Container 14 eine Steuereinrichtung 18 und diverse Aggregate (hier nicht im Detail dargestellt), wie eine oder mehrere Pumpen zum Austragen eines Dekontaminationsmittels, eine oder mehrere Pumpen zum Absaugen von kontaminiertem Abwasser aus dem Entwässerungsbereich, eine Fluidpumpe 19 zum Aufpumpen von Fluidkissen (Fig. 3), ein Stromaggregat u.a.. Der Container 16 beherbergt in diesem Ausführungsbeispiel einen oder mehrere Tanks mit Dekontaminationsmittel, Wasser und/oder zur Aufnahme des kontaminierten Abwassers. Das Dekontaminationsmittel kann Wasser mit oder ohne zusätzlichen Reinigungsmitteln und/oder chemischen Mitteln zum Entgiften, Entseuchen oder Entstrahlen sein. Dementsprechend kann ein patentgemäßes Dekontaminationssystem 10 prinzipiell auch zur "normalen" Reinigung von großformatigen Gegenständen verwendet werden.

Mit der Bezugsziffer 22 ist eine erste Sprühvorrichtung bezeichnet, die eine Vielzahl Sprühdüsen zum Austragen des Dekontaminationsmittels besitzt. Die erste Sprühvorrichtung beinhaltet hier ein Portal 26 mit zwei Säulen 28, die jeweils auf einem Laufwagen 30 abgestützt sind. Der Laufwagen besitzt Räder mit einer Bereifung (alternativ mit einer Kette), die es möglich machen, dass das Portal auf dem Boden bewegt werden kann, auf dem beispielsweise auch das Fahrzeug 12 fahren kann. Auf ein spezielles Schienensystem für das Portal kann in diesen Ausführungsbeispielen daher verzichtet werden.

In einigen bevorzugten Ausführungsbeispielen besitzt zumindest einer der Laufwagen 30 einen integrierten Antrieb 32, der es möglich macht, das Portal 26 relativ zu einem zu dekontaminierenden Fahrzeug 34 zu bewegen. In manchen Ausführungsbeispielen besitzt jeder der zwei Laufwagen 30 einen integrierten Antrieb 32, wobei die Antriebe gemeinsam von der Steuereinrichtung 18 angesteuert werden.

In den bevorzugten Ausführungsbeispielen kann das Portal 26 sowohl in der Höhe als auch in der Breite verstellt werden. Vorteilhaft werden sämtliche Bewegungen des Portals 26 von der Steuereinrichtung 18 automatisiert gesteuert. In einigen bevorzugten Ausführungsbeispielen ist die Steuereinrichtung 18 dementsprechend dazu eingerichtet, die Höhe und Breite des Portals 26 in Abhängigkeit von dem zu dekontaminierenden Fahrzeug 34 zu variieren, um den Abstand der an dem Portal 26 angeordneten Sprühdüsen 24 zu dem Fahrzeug auf einen definierten Wert einzustellen. Vorteilhaft sind die Sprühdüsen 24 an dem Portal 26 verschwenkbar angeordnet und die Steuereinrichtung 18 ist dazu eingerichtet, die Sprühdüsen 24 beim Austragen des Dekontaminationsmittels gezielt zu verschwenken, um eine vollständige Benetzung der Fahrzeugoberfläche mit dem Dekontaminationsmittel zu erreichen. Das Portal kann weitere Düsen (hier nicht dargestellt) zum Trocknen des Fahrzeugs 34 besitzen.

Abweichend von diesem Ausführungsbeispiel kann das Dekontaminationssystem 10 in anderen Ausführungsbeispielen ein feststehendes Portal 26 mit Sprühdüsen 24 beinhalten, wobei ein zu dekontaminierendes Fahrzeug relativ zu dem Portal gefahren werden muss. Des Weiteren könnten Sprühdüsen 24 in weiteren Ausführungsbeispielen an einem mehrachsig beweglichen Kranarm und/oder an einem oder mehreren mehrachsigen Roboterarmen angeordnet sein, um das Fahrzeug 34 automatisiert mit Dekontaminationsmittel zu benetzen. Das Dekontaminationsmittel (hier nicht dargestellt) kann eine wässrige oder aufgeschäumte Lösung mit einer geeigneten Chemikalie zum Entstrahlen, Entseuchen oder Entgiften sein. Beispielhaft wird auf die von der Kärcher Futuretech GmbH, Deutschland, angebotenen Dekontaminationsmittel GDS2000 zum Entgiften, BDS2000 zum Entseuchen und RDS2000 zum Entstrahlen verwiesen.

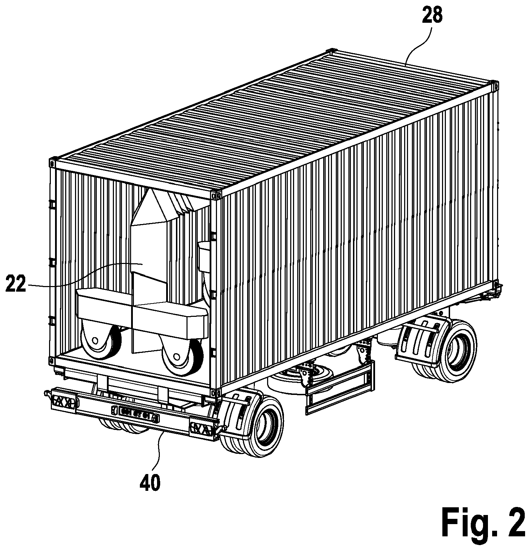

Fig. 2 zeigt einen Transportcontainer 38 gemäß ISO 668, der auf einem Fahrgestell 40 befestigt ist. Das Fahrgestell 40 ist hier ein Anhänger, der zum Beispiel an das Transportfahrzeug 12 angehängt werden kann. In dem Container 38 ist die erste Sprühvorrichtung 22 in einem Transportzustand dargestellt. Des Weiteren kann der Transportcontainer 38 die weiteren nachfolgend erläuterten Komponenten des Dekontaminationssystems 10, wie etwa die Wanne und eine Unterbodensprühvorrichtung, für den Transport zum Einsatzort beherbergen.

In Fig. 3 ist das zu dekontaminierende Fahrzeug 34 beim Einfahren in eine Wanne 42 dargestellt. In den bevorzugten Ausführungsbeispielen wird das Portal 26 über der Wanne 42 angeordnet, so dass das Portal 26 relativ zu der Wanne in Längsrichtung verfahren werden kann, um das Fahrzeug 34 mit Dekontaminationsmittel zu besprühen. Wie oben schon angedeutet, kann das Dekontaminationsmittel Wasser mit oder ohne zusätzlichen Reinigungsmitteln, wie etwa fettlösenden Mitteln und/oder schaumerzeugenden Mitteln sein. Des Weiteren kann das Dekontaminationsmittel Wasser mit chemischen Mitteln zum Entgiften, Entseuchen oder Entstrahlen sein oder auch reines Wasser zum Abspülen eines Fahrzeugs 34 sein.

Die Wanne 42 besitzt ein erstes Ende 44, an dem das Fahrzeug 34 in die Wanne 42 hineinfährt, und ein zweites Ende 46, an dem es die Wanne 42 in Längsrichtung 48 wieder verlassen kann, nachdem es mithilfe des Portals 26 und einer nachfolgend noch erläuterten Unterbodensprühvorrichtung dekontaminiert wurde. In allen bevorzugten Ausführungsbeispielen ist die Wanne 42 einteilig und sie besitzt eine Breite (orthogonal zu der Längsrichtung 48 gemessen), die größer ist als die maximale Breite der zu dekontaminierenden Fahrzeuge 34.

In dem dargestellten Ausführungsbeispiel besitzt die Wanne eine vorzugsweise einteilige aufrollbare oder zusammenlegbare Bodenplane 50 mit einem umlaufenden Wulst 52, der mit Hilfe der Fluidpumpe 19 mit Luft oder einem anderen Fluid (Gas oder Flüssigkeit) gefüllt werden kann, um zusammen mit der Bodenplane 50 die Wanne auszubilden. Am Einfahrende 44 besitzt die Wanne 42 hier eine seitliche Ausbuchtung, die einen definierten Entwässerungsbereich 54 für das in der Wanne 42 gesammelte Abwasser bildet. Wie in Fig. 3 dargestellt ist, umgibt die Wulst 52 in diesem Ausführungsbeispiel auch den Entwässerungsbereich 54, so dass das Abwasser bis zur gezielten Absaugung an einer Entnahmestelle 56 vollständig in der Wanne 42 gehalten wird.

In einigen Ausführungsbeispielen besitzt der Entwässerungsbereich 54 ein Bodenniveau, das niedriger liegt als das Bodenniveau der Wanne 42 in dem Behandlungsbereich 58 in Längsrichtung 48 zwischen den beiden Enden 44, 46. Das abgesenkte Bodenniveau des Entwässerungsbereichs 54 ist in Fig. 3 mit einer Stufe 60 angedeutet.

In einigen, hier nicht dargestellten Ausführungsbeispielen kann die Wanne quer zu der Längsrichtung 48 eine konvexe Wölbung besitzen, die sich in Längsrichtung erstreckt. In diesen Ausführungsbeispielen besitzt die Wanne somit in Längsrichtung verlaufende, seitliche Vertiefungen, in denen sich das Abwasser aus dem Behandlungsbereich 58 zunächst sammeln kann, bevor es dem definierten Entwässerungsbereich 54 zugeführt wird. In diesen Ausführungsbeispielen besitzt das neue Dekontaminationssystem vorteilhaft zwei definierte Entwässerungsbereiche 54, die auf gegenüberliegenden Seiten von dem Anfangsbereich 44 angeordnet sind.

Bei der Bezugsziffer 62 sind eine Vielzahl Fluidkissen dargestellt, die hier vom zweiten Ende 46 entgegen der Längsrichtung 48 in der Wanne 42 angeordnet sind. In einigen Ausführungsbeispielen sind die Fluidkissen 62 integraler Bestandteil der Bodenplane 50. Sie können beispielsweise in eine mehrlagige Bodenplane eingearbeitet und/oder mit der Bodenplane 50 verschweißt sein. In anderen Ausführungsbeispielen können die Fluidkissen 62 in die Wanne 42 eingelegt sein und beispielsweise mit Spanngurten gegen Verrutschen gesichert sein.

Die Fluidkissen 62 sind hier Bestandteil eines Wellenerzeugers, der dazu eingerichtet ist, das in der Wanne 42 befindliche Abwasser in eine Wellenbewegung entgegen der Längsrichtung 48 zu versetzen. Vorteilhaft ist die Steuereinrichtung 18 dazu eingerichtet, die Fluidkissen mit Hilfe der Fluidpumpe 19 in einer definierten Abfolge stoßweise aufzublasen und wieder zu entlüften, um auf diese Weise eine Wellenbewegung mit einer definierten Wellenlaufrichtung 64 hervorzurufen. Das in der Wanne 42 befindliche Abwasser wird aufgrund der Wellenbewegung aktiv zu dem ersten Ende 44 befördert und sammelt sich dort in dem Entwässerungsbereich 54, von wo aus es definiert abgesaugt werden kann. Aufgrund der aktiv erzeugten Wellenbewegung 64 werden auch größere Schlammklumpen zerkleinert, abgetragen, mitgerissen und schließlich zu dem Entwässerungsbereich 54 befördert.

In einigen vorteilhaften Ausführungsbeispielen besitzt das Dekontaminationssystem 10 im Bereich der Wanne 42 Strahldüsen 66, über die ein Wasserstrahl gezielt in Richtung des ersten Endes 44 eingespeist werden kann. Die mithilfe der Strahldüsen 66 erzeugten Wasserstrahlen unterstützen die Transportbewegung des Abwassers durch eine definierte Strömungsrichtung.

In dem Entwässerungsbereich 54 kann vorteilhaft ein Rotor 68 angeordnet sein, der größere Schlammklumpen zerteilt und Sedimente in dem Entwässerungsbereich 54 aufwirbelt, damit sie an der Entnahmestelle 56 effizient abgesaugt werden können.

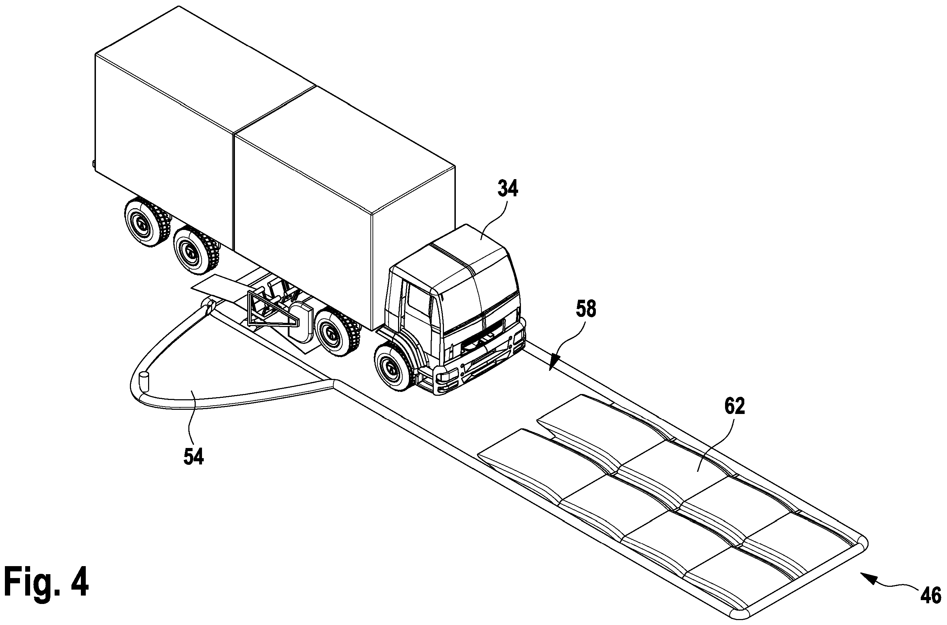

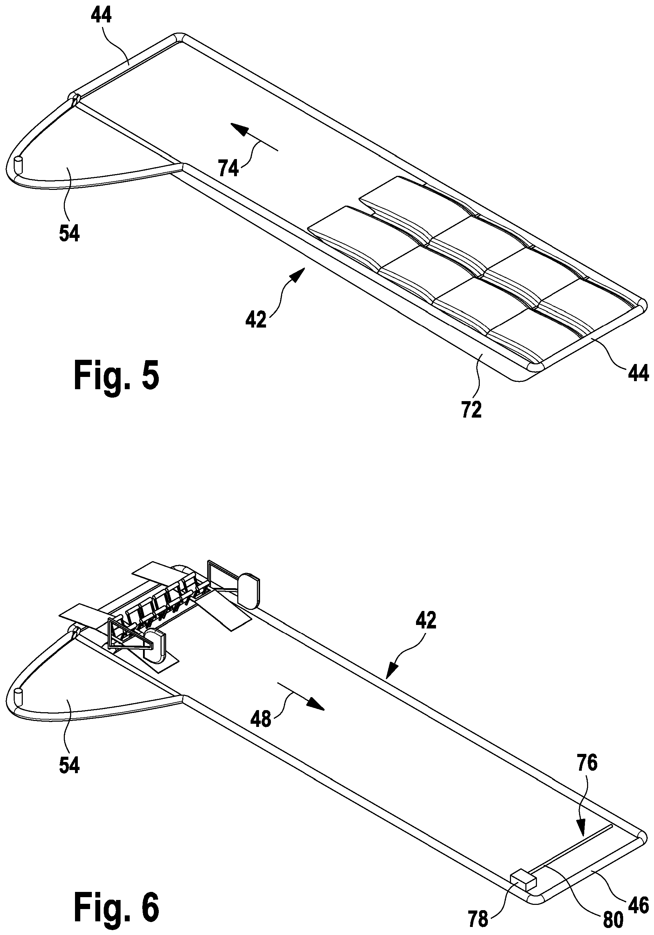

Abweichend von der Darstellung in Fig. 3 und Fig. 4 kann die Wanne 42 in weiteren Ausführungsbeispielen einen zweiten Entwässerungsbereich am linksseitigen Wannenende und/oder unterhalb der im Folgenden näher erläuterten Unterbodensprühvorrichtung 70 besitzen. Außerdem kann die Wanne 42, wie in Fig. 5 angedeutet ist, alternativ oder ergänzend eine Keilstruktur 72 aufweisen, die dazu eingerichtet ist, das zweite Ende 46 relativ zu dem ersten Ende 44 anzuheben und somit ein definiertes Gefälle in Richtung des Pfeils 74 zu bewirken. Das Gefälle lässt das in der Wanne gesammelte Abwasser definiert in Richtung des Entwässerungsbereichs 54 abfließen.

In weiteren Ausführungsbeispielen kann die Wanne 42 einen Wellenerzeuger besitzen, der im Wesentlichen eine Longitudinalwelle erzeugt. In Fig. 6 ist ein solcher Wellenerzeuger an dem zweiten Ende 46 dargestellt und mit der Bezugsziffer 76 bezeichnet. Der Wellenerzeuger 76 besitzt hier einen - vorzugsweise elektrischen - Motor 78, der ein Oszillationselement 80 antreibt. Das Oszillationselement 80 ist hier ein Balken, der in der Wanne 42 angeordnet ist und quer zu der Längsrichtung 48 verläuft. Eine oszillierende Hin- und Herbewegung des Balkens entgegen der Längsrichtung 48 erzeugt eine Longitudinalwelle in dem Abwasser, die entgegen der Längsrichtung 48 läuft.

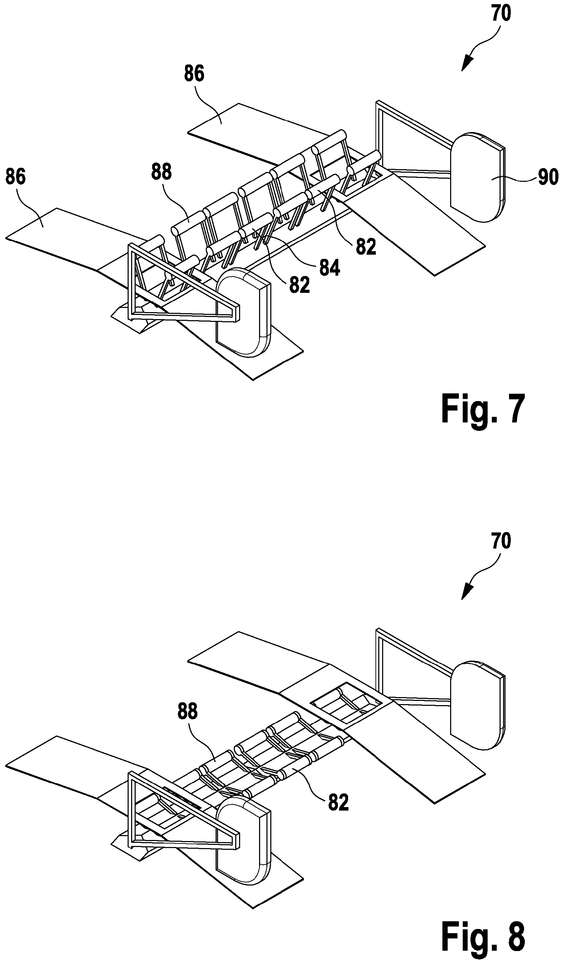

In den Fig. 7 und 8 ist ein Ausführungsbeispiel einer vorteilhaften Unterbodensprühvorrichtung 70 in zwei verschiedenen Betriebsstellungen dargestellt. Die Unterbodensprühvorrichtung 70 besitzt eine Vielzahl von Unterbodensprühdüsen 82, die hier an einem federnd gelagerten Scherenmechanismus 84 angeordnet sind. Der Scherenmechanismus 84 macht es möglich, die Sprühdüsen 82 in vertikaler Richtung anzuheben (Fig. 7) oder abzusenken (Fig. 8), um den Sprühabstand zwischen einer Sprühdüse 82 und dem Unterboden des Fahrzeugs 34 einzustellen. In einigen Ausführungsbeispielen besitzt die Unterbodensprühvorrichtung 70 eine Vielzahl von Unterbodensprühdüsen 82, die quer zu der Längsrichtung 48 der Wanne 42 nebeneinander angeordnet sind und so miteinander gekoppelt sind, dass sie sich in vertikaler Richtung gemeinsam nach oben oder nach unten bewegen, wie dies in Fig. 8 dargestellt ist. In anderen Ausführungsbeispielen können die nebeneinander angeordneten Unterbodensprühdüsen 82 individuell in der vertikalen Höhe verstellt werden, so dass beispielsweise die in Fig. 7 und 8 jeweils außenliegenden Unterbodensprühdüsen beim Überfahren mit dem Fahrzeug 34 heruntergedrückt werden, während die dazwischenliegenden Unterbodensprühdüsen 82 in ihrer oberen Stellung verbleiben, um den parallel zur Fahrzeugachse liegenden Fahrzeugunterboden aus geringer Entfernung zu besprühen.

Vorteilhaft besitzt die Unterbodensprühvorrichtung 70 in einigen Ausführungsbeispielen zwei Rampen 86, die das Überfahren der Unterbodensprühvorrichtung 70 mit dem Fahrzeug 34 erleichtern. Wie in den Fig. 7 und 8 dargestellt ist, können Unterbodensprühdüsen 82 in einer geeigneten Ausnehmung der Rampen 80 angeordnet sein, um sowohl die Fahrzeugräder als auch den in Längsrichtung zwischen den Fahrzeugrädern liegenden Unterbodenbereich mit Dekontaminationsmittel zu benetzen.

In einigen Ausführungsbeispielen besitzt die Unterbodensprühvorrichtung 70 ein oder mehrere Abtastelemente, beispielsweise in Form einer an dem Scherenmechanismus angeordneten Rolle 88, mit denen der Fahrzeugunterboden abgetastet werden kann. Sobald das Fahrzeug 34 eine Rolle 88 nach unten drückt, bewegt sich die hier mechanisch mit der Rolle 88 gekoppelte Sprühdüse 82 ebenfalls nach unten.

In weiteren Ausführungsbeispielen kann die Unterbodensprühvorrichtung 70 ein oder mehrere in Längsrichtung 48 vorlaufende und ein oder mehrere in Längsrichtung 48 nachlaufende Abtastelemente besitzt. Vorteilhaft sind eine oder mehrere Sprühdüsen dann in Längsrichtung zwischen den vorlaufenden und nachlaufenden Abtastelementen angeordnet. Der Unterboden des Fahrzeugs kann so noch genauer abgetastet werden und die eine oder mehrere Sprühdüsen können in Abhängigkeit von den Abtastelementen um eine Achse quer zu der Längsrichtung 48 verschwenkt werden. In einigen Ausführungsbeispielen erfolgt dieses verschwenken mit Hilfe des federnd gelagerten Scherenmechanismus 84 ohne zusätzliche elektrische Antriebe für die Schwenkbewegung.

Vorteilhaft besitzt die Unterbodensprühvorrichtung 70 in den bevorzugten Ausführungsbeispielen zusätzliche seitliche Sprühvorrichtungen 90, die in Bodennähe angeordnet sind und insbesondere dazu eingerichtet sind, bodennahe Fahrzeugbereiche, wie beispielsweise die Radfelgen und die seitlichen Reifenflanken, mit Dekontaminationsmittel zu benetzen. Alternativ oder ergänzend können derartige Seitensprühvorrichtungen an dem Portal 28 angeordnet sein.

Abweichend von der Darstellung in den Fig. 7 und 8 kann die Unterbodensprühvorrichtung in weiteren Ausführungsbeispielen selbstfahrend sein und dementsprechend einen Antrieb besitzen, so dass die Unterbodensprühvorrichtung automatisiert und mit einer definierten Geschwindigkeit relativ zu dem zu dekontaminierenden Fahrzeug bewegt werden kann. Sämtliche Details der Unterbodensprühvorrichtung sind auch für sich genommen und ohne den separaten Entwässerungsbereich der Wanne eine vorteilhafte Weiterbildung des neuen Dekontaminationssystems gegenüber bekannten Systemen.

In allen bevorzugten Ausführungsbeispielen ist das neue Dekontaminationssystem dazu eingerichtet, einen definierten Dekontaminationsprozess automatisiert und mit geringem Personalbedarf sowie verbrauchsmittelsparsam durchzuführen. Vorteilhaft wird das von dem zu dekontaminierenden Fahrzeug ablaufende Dekontaminationsmittel vollständig in der Wanne gesammelt und über einen abgesetzten Entwässerungsbereich in der Wanne abgesaugt. Um auch große Schlammklumpen dem Entwässerungsbereich gezielt zuzuführen, besitzt das Dekontaminationssystem vorteilhaft eine Anordnung, die das Abwasser dem Entwässerungsbereich gezielt und gerichtet zuführt.

## Patentansprüche

1. Mobiles Dekontaminationssystem zum Entstrahlen, Entseuchen oder Entgiften von Fahrzeugen (34), mit zumindest einer Sprühvorrichtung (22) zum gezielten Austragen eines zumindest teilweise flüssigen Dekontaminationsmittels auf das Fahrzeug (34), und mit einer Steuereinrichtung (18), die dazu eingerichtet ist, die zumindest eine Sprühvorrichtung (22) in Abhängigkeit von dem zu dekontaminierenden Fahrzeug (34) automatisiert zu betreiben, **gekennzeichnet durch** eine Wanne (42), in der das Fahrzeug (34) beim Austragen des Dekontaminationsmittels positioniert werden kann, um das vom Fahrzeug (34) ablaufende Dekontaminationsmittel zu sammeln, wobei die Wanne (42) ein erstes Ende (44) und ein zweites Ende (46) besitzt und eine Längsrichtung (48) mit einem Anfangsbereich an dem ersten Ende (44), einem Behandlungsbereich (58) für das Fahrzeug (34) und einem Endbereich an dem zweiten Ende (46) definiert, wobei ein zu dekontaminierendes Fahrzeug (34) an dem ersten Ende (44) in die Wanne (42) hineinfahren kann, wobei sich das zu dekontaminierende Fahrzeug (34) während der Dekontamination in dem Behandlungsbereich (58) befindet, und wobei das Fahrzeug (34) die Wanne an dem zweiten Ende (46) wieder verlässt, wobei die zumindest eine Sprühvorrichtung (22) eine Unterbodensprühvorrichtung (70) beinhaltet, die an dem ersten Ende (44) in der Wanne (42) angeordnet ist, wobei die Wanne (42) eine umlaufende Wulst (52) und eine seitliche Ausbuchtung besitzt, die einen definierten Entwässerungsbereich (54) für das in der Wanne (42) gesammelte Abwasser bildet, und wobei der definierte Entwässerungsbereich (54) in der Nähe des Anfangsbereichs (44) angeordnet und somit von dem Behandlungsbereich (58) separiert ist, und ferner **gekennzeichnet durch** eine Anordnung, die dazu eingerichtet ist, das Dekontaminationsmittel in der Wanne (42) dem definierten Entwässerungsbereich (54) gerichtet zuzuführen.

2. Mobiles Dekontaminationssystem nach Anspruch 1, **dadurch gekennzeichnet, dass** die Anordnung einen Wellenerzeuger (62; 76) aufweist, der dazu eingerichtet ist, das in der Wanne (42) gesammelte Dekontaminationsmittel in eine Wellenbewegung mit einer definierten Wellenlaufrichtung (64) zu versetzen, wobei die definierte Wellenlaufrichtung (64) zu dem Entwässerungsbereich (54) gerichtet ist.

3. Mobiles Dekontaminationssystem nach Anspruch 2, **dadurch gekennzeichnet, dass** der Wellenerzeuger zumindest ein Fluidkissen (62) aufweist, das in oder unter der Wanne (42) angeordnet ist, und eine Fluidpumpe (19), die dazu eingerichtet ist, das zumindest eine Fluidkissen (62) wahlweise aufzupumpen und/oder zu entleeren.

4. Mobiles Dekontaminationssystem nach Anspruch 2 oder 3, **dadurch gekennzeichnet, dass** der Wellenerzeuger eine Vielzahl an Fluidkissen (62) aufweist, die in einer Reihe zueinander angeordnet sind.

5. Mobiles Dekontaminationssystem nach einem der Ansprüche 2 bis 4, **dadurch gekennzeichnet, dass** der Wellenerzeuger (76) einen Motor (78) aufweist, der ein Oszillationselement (80) antreibt.

6. Mobiles Dekontaminationssystem nach einem der Ansprüche 1 bis 5, **dadurch gekennzeichnet, dass** die Anordnung Strahldüsen (66) aufweist, die dazu eingerichtet sind, das gesammelte Dekontaminationsmittel dem definierten Entwässerungsbereich (54) mit Hilfe von Fluidstrahlen zuzuführen.

7. Mobiles Dekontaminationssystem nach einem der Ansprüche 1 bis 6, **dadurch gekennzeichnet, dass** die Wanne (42) einen Wannenboden (50) aufweist und dass die Anordnung eine Keilstruktur (72) aufweist, die dazu eingerichtet ist, den Wannenboden (50) mit einer definierten Ablaufrichtung zu dem definierten Entwässerungsbereich (54) schräg zu stellen.

8. Mobiles Dekontaminationssystem nach einem der Ansprüche 1 bis 7, **dadurch gekennzeichnet, dass** die Steuereinrichtung (18) ferner dazu eingerichtet ist, das Dekontaminationsmittel in der Wanne (42) dem definierten Entwässerungsbereich (54) automatisiert zuzuführen.

9. Mobiles Dekontaminationssystem nach einem der Ansprüche 1 bis 8, **dadurch gekennzeichnet, dass** die zumindest eine Sprühvorrichtung (22) ein fahrbares Portal (26) mit zwei Säulen (28) aufweist, die jeweils auf einem Laufwagen (30) abgestützt sind.

10. Mobiles Dekontaminationssystem nach Anspruch 9, **dadurch gekennzeichnet, dass** zumindest ein Laufwagen einen Antrieb (32) besitzt.

11. Mobiles Dekontaminationssystem nach einem der Ansprüche 1 bis 10, **dadurch gekennzeichnet, dass** die Unterbodensprühvorrichtung (70) zumindest eine Unterbodensprühdüse (82) aufweist, die in vertikaler Richtung verstellbar ist, um einen definierten Sprühabstand zwischen der zumindest einen Unterbodensprühdüse (82) und einem Fahrzeugunterboden einzustellen.

## Claims

1. A mobile decontamination system for radiological decontamination, disinfection and/or detoxification of vehicles (34), comprising at least one spraying device (22) for the selective discharge of an at least partially liquid decontaminant onto the vehicle (34), and comprising a control device (18) which is configured to operate the at least one spraying device (22) in an automated manner as a function of the vehicle (34) to be decontaminated, **characterized by** a trough (42) in which the vehicle (34) can be positioned as the decontaminant is discharged to collect the decontaminant draining from the vehicle (34), wherein the trough (42) has a first end (44) and a second end (46) and defines a longitudinal direction (48) with an entry area at the first end (44), a treatment area (58) for the vehicle (34), and an exit area at the second end (46), wherein a vehicle (34) to be decontaminated can enter the trough (42) at the first end (44), wherein the vehicle (34) to be decontaminated is in the treatment area (58) during decontamination, and wherein the vehicle (34) exits the trough at the second end (46), wherein the at least one spraying device (22) includes an underbody spraying device (70) disposed in the trough (42) at the first end (44), wherein said trough (42) has a circumferential bead (52) and a lateral bulge defining a defined drainage area (54) for polluted water collected in said trough (42), wherein said defined drainage area (54) is located proximate said entry area (44) and thus is separated from said treatment area (58), and further **characterized by** an arrangement adapted to direct decontaminant in said trough (42) to said defined drainage area (54).

2. The mobile decontamination system according to claim 1, **characterized in that** the arrangement comprises a wave generator (62; 76) configured to impart a wave motion with a defined wave travel direction (64) to the decontaminant collected in the trough (42), wherein the defined wave travel direction (64) is directed towards the drainage area (54).

3. The mobile decontamination system according to claim 2, **characterized in that** the wave generator comprises at least one fluid pad (62) disposed in or below the trough (42), and comprises a fluid pump (19) configured to selectively inflate and/or deflate the at least one fluid pad (62).

4. The mobile decontamination system according to claim 2 or 3, **characterized in that** the wave generator comprises a plurality of fluid pads (62) arranged in a row with respect to each other.

5. The mobile decontamination system according to any one of claims 2 to 4, **characterized in that** the wave generator (76) comprises a motor (78) driving an oscillating element (80).

6. The mobile decontamination system according to any one of claims 1 to 5, **characterized in that** the arrangement comprises jet nozzles (66) configured to drive the collected decontaminant to the defined drainage area (54) by means of fluid jets.

7. The mobile decontamination system according to any one of claims 1 to 6, **characterized in that** the trough (42) comprises a trough bottom (50) and **in that** the arrangement comprises a wedge structure (72) configured to incline the trough bottom (50) with a defined drainage direction towards the defined drainage area (54).

8. The mobile decontamination system according to any one of claims 1 to 7, **characterized in that** the control device (18) is further configured to automatically drive the decontaminant in the trough (42) to the defined drainage area (54).

9. The mobile decontamination system according to any one of claims 1 to 8, **characterized in that** the at least one spraying device (22) comprises a mobile gantry (26) having two columns (28) each supported on a carriage (30).

10. The mobile decontamination system according to claim 9, **characterized in that** at least one carriage has a drive (32).

11. The mobile decontamination system according to any one of claims 1 to 10, **characterized in that** the underbody spraying device (70) comprises at least one underbody spray nozzle (82) that is vertically adjustable to set a defined spray distance between the at least one underbody spray nozzle (82) and a vehicle underbody.

## Revendications

1. Système de décontamination mobile destiné à décontaminer, désinfecter ou désintoxiquer des véhicules (34), ledit système comprenant au moins un dispositif de pulvérisation (22) destiné à l'application ciblée d'un agent de décontamination au moins partiellement liquide sur le véhicule (34) et un dispositif de commande (18) qui est conçu pour actionner automatiquement l'au moins un dispositif de pulvérisation (22) en fonction du véhicule (34) à décontaminer, **caractérisé par** une cuve (42) dans laquelle le véhicule (34) peut être positionné lors de la délivrance de l'agent de décontamination afin de récupérer l'agent de décontamination dégoulinant du véhicule (34), la cuve (42) possédant une première extrémité (44) et une deuxième extrémité (46) et définissant une direction longitudinale (48) comportant une zone initiale à la première extrémité (44), une zone de traitement (58) destinée au véhicule (34) et une zone finale à la deuxième extrémité (46), un véhicule (34) à décontaminer pouvant entrer dans la cuve (42) à la première extrémité (44), le véhicule (34) à décontaminer se trouvant dans la zone de traitement (58) pendant la décontamination, et le véhicule (34) quittant à nouveau la cuve à la deuxième extrémité (46), l'au moins un dispositif de pulvérisation (22) contenant un dispositif de pulvérisation de dessous de caisse (70) qui est disposé à la première extrémité (44) dans la cuve (42), la cuve (42) possédant un bourrelet circonférentiel (52) et un renflement latéral qui forme une zone de drainage définie (54) pour les eaux usées collectées dans la cuve (42), et la zone de drainage définie (54) étant disposée à proximité de la zone initiale (44) et étant ainsi séparée de la zone de traitement (58), et **caractérisée en outre par** un ensemble qui est conçu pour amener l'agent de décontamination dans la cuve (42) en le dirigeant vers la zone de drainage définie (54).

2. Système de décontamination mobile selon la revendication 1, **caractérisé en ce que** l'ensemble comporte un générateur d'ondes (62 ; 76) qui est conçu pour conférer à l'agent de décontamination collecté dans la cuve (42) un mouvement ondulatoire avec une direction d'ondulation définie (64), la direction d'ondulation définie (64) étant dirigée vers la zone de drainage (54).

3. Système de décontamination mobile selon la revendication 2, **caractérisé en ce que** le générateur d'ondes comporte au moins un coussin de fluide (62) qui est disposé dans ou sous la cuve (42) et une pompe à fluide (19) qui est conçue pour au choix gonfler et/ou vider l'au moins un coussin de fluide (62).

4. Système de décontamination mobile selon la revendication 2 ou 3, **caractérisé en ce que** le générateur d'ondes comporte un grand nombre de coussins de fluide (62) qui sont disposés en rangée les uns par rapport aux autres.

5. Système de décontamination mobile selon l'une des revendications 2 à 4, **caractérisé en ce que** le générateur d'ondes (76) comporte un moteur (78) qui entraîne un élément oscillant (80).

6. Système de décontamination mobile selon l'une des revendications 1 à 5, **caractérisé en ce que** l'ensemble comporte des buses à jet (66) qui sont conçues pour amener l'agent de décontamination collecté à la zone de drainage définie (54) à l'aide de jets de fluide.

7. Système de décontamination mobile selon l'une des revendications 1 à 6, **caractérisé en ce que** la cuve (42) comporte un fond de cuve (50) et **en ce que** l'ensemble comporte une structure en coin (72) qui est conçue pour placer le fond de cuve (50) obliquement par rapport à la zone de drainage définie (54) avec une direction de drainage définie.

8. Système de décontamination mobile selon l'une des revendications 1 à 7, **caractérisé en ce que** le dispositif de commande (18) est en outre conçu pour amener automatiquement l'agent de décontamination à la zone de drainage définie (54) dans la cuve (42).

9. Système de décontamination mobile selon l'une des revendications 1 à 8, **caractérisé en ce que** l'au moins un dispositif de pulvérisation (22) comporte un portique mobile (26) pourvu de deux colonnes (28) qui prennent appui chacune sur un chariot (30).

10. Système de décontamination mobile selon la revendication 9, **caractérisé en ce qu'**au moins un chariot comporte un entraînement (32).

11. Système de décontamination mobile selon l'une des revendications 1 à 10, **caractérisé en ce que** le dispositif de pulvérisation de dessous de caisse (70) comporte au moins une buse de pulvérisation de dessous de caisse (82) qui est déplaçable dans la direction verticale afin de régler une distance de pulvérisation définie entre l'au moins une buse de pulvérisation de dessous de caisse (82) et le dessous de caisse d'un véhicule.
